# EUROPEAN PATENT APPLICATION

(11) **EP 4 759 247 A1**
(43) Date of publication of application: **17.06.2026**
(21) Application number: 24307112.3
(22) Date of filing: 13.12.2024
(51) Int. Cl.: A61B 17/17, A61B 17/88

(54) **SYNCHRONIZED SURGICAL GUIDE**

(71) Applicant: Extremis Robotics, 38400 Saint-Martin-D'Hères (FR)
(72) Inventor: GERLAND, Hugo, 38400 Saint-Martin-d'Hères (FR)
(74) Representative: Icosa

(57) **Abstract**

The present invention relates to a surgical guide for moving a second bone portion between a pathological position and an adjusted position with respect to a first bone portion, during osteosynthesis surgery. The invention is characterized in that securing assembly is connected to the first guiding assembly by a coupling member configured to simultaneously move the securing assembly to modify the fixing angle and the first guiding assembly so that a displacement of the second bone portion along the longitudinal axis implies a modification of the fixing angle, ensuring the fixing wire can traverse both the first bone portion and the second bone portion after a displacement of the first guiding assembly.

## Description

### FIELD OF INVENTION

The present invention pertains to the field of osteosynthesis surgery. More precisely, the invention relates to a surgical guide for moving a second bone portion between a pathological position and an adjusted position with respect to a first bone portion, during osteosynthesis surgery. The invention further relates to the corresponding method.

### BACKGROUND OF INVENTION

Osteosynthesis refers to a surgical technique in the field of orthopaedics where two or more bone fragments are joined together using mechanical devices like screws, nails, or plates to stabilize them until the bone heals as a single unit. Osteosynthesis surgery are required after osteotomy, or to treat bone fractures. Osteosynthesis surgery is one of the different surgical techniques that allow to correct as example Hallux Valgus deformation, including open surgical procedures and minimally invasive surgical procedure (MIS).

Hallux-valgus is a progressive distortion of the metatarsophalangeal (MTP) joint of the large toe (Hallux), which deforms outwards (valgus) the first metatarsal bone. This deformation of the phalanx may also be called "Bunion" due to the bony prominence on the medial aspect of the foot It often results from biomechanical imbalances, genetic predisposition, or improper footwear, causing misalignment of the sesamoid bones and abnormal stress on surrounding soft tissues, including the joint capsule and ligaments. Over time, this misalignment may lead to pain, inflammation, and functional impairment of the forefoot or lower limb, due to cramps or bad knee extensions, and even to the hip or back.

MIS procedures present several advantages over open procedure, mostly because of post-operative benefits, were some of those benefits include less pain, a decreased joint stiffness, a possibility to bear body weight earlier, smaller scars, a decreased operative time, and a reduced risk of infection or wound complications.

MIS procedures comprise several steps including the osteotomy of the first metatarsal into two bone portions to reduce the angle between the first metatarse and the first proximal phalange. The two bone portions obtained from osteotomy of the first metatarsal are the first bone portion, referring to the bone portion in relation to the medial cuneiform bone, and the second bone portion referring to the metatarsal head involved in the metatarsophalangeal joint.

Standard techniques involve an inward lateral translation of the metatarsal head in order to reduce the hallux valgus angle and adjust the overall bone portions position. Standard techniques consist then in the stabilization of the two bone portions of the first metatarsal bone by joining the at least two bone portions and secure them together using mechanical devices like screws, nails, plates or wires to stabilize them until the bone heals as a single unit.

Surgeon's gestures and know-how plays a major role in hallux valgus surgery. Following the use of surgical guide to promote an inward lateral translation of the bone portion comprising the first metatarsal bone head, it is also necessary to adjust fixing system insertion to secure the first bone portion with the second bone portion in an adjusted position.

State of the art solution involve surgical guides having multiple independent controlling systems to adjust the position of each part of the surgical guide.

Due to the complexity of use of existing devices, but also due to the mechanical constraints applied by tissues, tendons and joints on the second bone portion, it may be difficult to properly secure the two bone portions together. The main consequences involve imperfect bone portions fixation, post-surgeries malunions of bone portions, and musculoskeletal pain even after treatment of Hallux valgus. Those musculoskeletal pains can include backpain, knee pain, ankle pain or any other trauma due to compensatory posture changes between the spine and lower extremities. Worse cases can lead to shortening, elevation, plantarflexion, varus/valgus, and rotational of the first metatarsal. Those incomplete recovery after surgery can also cause a developing Metatarsophalangeal osteoarthritis.

It is thus an objective of the invention to overcome the disadvantages of existing solutions by providing a surgical guide enabling to solve the technical problem of finding an aiming solution for surgical guide that ensures proper bone fixation while simplifying the use for surgeons. Providing a surgical guide enabling that eases the securing step of the bone portions will thus provide surgeons with an improved solution for precision bone portion position adjustment. Thus, it will lead to greater patient recovery post-surgery and avoid post-surgical complications.

### SUMMARY

This invention thus relates to a surgical guide for moving a second bone portion between a pathological position and an adjusted position with respect to a first bone portion, during osteosynthesis surgery, the surgical guide being configured to be positioned in a surgical plane of the first bone portion and the second bone portion, said surgical guide comprising:
- a body with an upper extremity and a lower extremity, extending in a median plane, and comprising a bone anchoring member configured to be in contact with the first bone portion at an anchor point in the surgical plan, a securing assembly comprising at least one fixing wire, said at least one fixing wire being configured to be inserted into the first bone portion and the second bone portion to secure the first bone portion with the second bone portion, once the second bone portion is in the adjusted position, the fixing wire being inclined of a fixing angle with relation to a central axis of the first bone portion, the fixing angle being measured in the surgical plane between the fixing wire and the central axis of the first bone portion and, the central axis being tangential to the anchoring point, the fixing angle being adjustable between a first extremal position and a second extremal position in order to set the direction of the at least one fixing wire in relation to the second bone portion,
- an adjustment member comprising a first guiding assembly connected to the upper extremity of the body and moveable along a longitudinal axis of the median plane, wherein the first guiding assembly includes one positioning member configured to come in contact with the second bone portion in at least one contacting point to move the second bone portion along the longitudinal axis,
***characterized in that*** the securing assembly is connected to the first guiding assembly by a coupling member configured to simultaneously move the securing assembly between a first extremal position and a second extremal position to modify the fixing angle, and the first guiding assembly along the longitudinal axis, so that a displacement of the second bone portion along the longitudinal axis implies a modification of the fixing angle, ensuring the fixing wire can traverse both the first bone portion and the second bone portion after a displacement of the first guiding assembly.

In other words, the surgical guide according to the invention enables to simultaneously correct a second bone position in regard to a first bone position with an inward translation while adjusting a securing member enabling to obtain the required angle for insertion of fixing systems such as wires, and securing two bones portions together. This kinematic synchronization enables to simplify surgeons practice by eliminating a step of surgical guide adjustment (e.g., adjusting a securing assembly). This increased precision not only facilitates improved bones position securing but also reduces post-surgery risks, including pain and complications, thus contributing to better patient outcomes.

According to other aspects of the invention, the device comprises one or more of the features described in the following embodiments, taken alone or in any possible combination.

According to one embodiment the body extends from the upper extremity towards the lower extremity according to a curvature parallel to the surgical plane, and the securing assembly is movable along the curvature between the first extremal position and the second extremal position.

In other words, the design of the body enables to adjust the fixing angle within an amplitude predetermined by the geometry, including length and curvature radius.

According to one embodiment of the invention, the coupling member is configured to be locked when the first guiding assembly is not actuated.

In other words, the coupling device enables kinetical synchronization depending on the actuation of the first guiding assembly. It is not possible to trigger a translation of the guiding assembly by pushing or pulling the securing assembly. A movement of the securing assembly is only possible by actuation of the first guiding assembly, thus providing precision during surgical procedure.

According to one embodiment of the invention, the first guiding assembly comprises a translation screw threaded through a mechanical controller that is fixed to the body and wherein the assembling of the translation screw and the mechanical controller (221) is configured to present a tightening torque preventing from uncontrolled movements.

In other words, the design of the translation screw of the first guiding assembly enables to provide locking functions.

According to one embodiment of the invention, the coupling member allows the securing assembly to be reversibly adjusted, in a direction from the first extremal position to the second extremal position, and in a reversed direction from the second extremal position to the first extremal position.

In other words, the coupling member enables the user of the surgical guide to increase or decrease the fixing angle depending on the translation of the first guiding assembly. In case of pushing too far the second bone portion, surgeon can decide to go backward with the device without finding the surgical device blocked in a position.

According to one embodiment of the invention, the coupling member comprises a gear rack connected with the first guiding assembly and a mechanical transmission which is meshed with the gear rack, and which is configured to be connected with the securing assembly so that the fixing angle is adjusted according the meshing of the mechanical transmission when the gear rack moves.

In other words, mechanical components enable to transform the linear translation of the first guiding assembly in a curved motion to adjust the fixing angle. Using gear rack and mechanical transmission enables high precision up to the micrometric level based on gear step design.

According on embodiment of the invention, the mechanical transmission comprises a cam configured to face a pad linked to the securing assembly, when the securing assembly is in the first/second extremal position, and come into contact with the pad, when the securing assembly is in an intermediate position between the first extremal position and the second extremal position, and apply a pressure on the pad when the securing assembly is in the second/first extremal position.

According one embodiment of the invention, the cam of the mechanical transmission is in connection with the securing assembly through a connecting rod configured to adjust the fixing angle according to the mechanical transmission rotation.

In other words, mechanical components enable to transform the linear translation of the first guiding assembly in a curved motion to adjust the fixing angle. Using connecting rod to connect the securing assembly with the mechanical transmission enables high precision up to the micrometric level based on gear step design.

According to one embodiment of the invention, the protective pad is mounted on the connecting rod.

In other words, the protective pad is compressed during movements of the connecting rod, thus frictional forces against the cam will prevent any uncontrolled movement of the securing assembly.

According one embodiment of the invention the adjustment member further comprises a second guiding assembly comprising a first positioning element configured to come in contact with the second bone portion at a first contact site, the first positioning element being configured to move the contact site from an initial position in the surgical plane to a revised position comprised in a plane orthogonal to the body median plane and wherein in the revised position, the second bone portion is comprised outside the surgical plane.

In other words, the adjustment member enables a multidirectional positioning of the second bone portion using a first guiding assembly to provide a translation in the surgical plan while the second guiding assembly enables to project the second bone portion outside of the surgical plan with a movement in z-axis direction. Specifically, it allows precise adjustments transverse to the median plane of the foot in case of hallux valgus surgery. This increased precision improves bones portions alignment and also reduces post-surgery risks, including pain and complications, thus contributing to better patient outcomes.

According to one embodiment, the first guiding assembly comprises a carriage movable along the first guiding assembly longitudinal axis, along a guide formed in the body, said carriage comprising said at least one positioning member.

In other words, the translation of the second bone element in the surgical plane is obtained thanks to a carriage supporting at least one positioning member. Several configurations are possible.

According to a first configuration, the first guiding assembly comprises a first positioning member mounted on a lateral wall of the carriage, said first positioning member being configured to push said second bone portion along the first guiding assembly longitudinal axis.

In other words, the surgical guide may comprise a single positioning member whose only function is to push the second bone portion in the surgical plane.

According to a second configuration, the first guiding assembly comprises a positioning member holder mounted on the carriage, said positioning member holder comprising a second positioning member configured to push and pull the second bone portion along the first guiding assembly longitudinal axis.

In this configuration, the surgical guide may comprise a single positioning member configured to both push and pull the second bone portion in the surgical plane.

According to a third configuration, the surgical guide may comprise two positioning members, including the first positioning member configured to push the second bone portion and the second positioning member configured to either pull the second bone portion or both push and pull the second bone portion in the surgical plane.

According to one embodiment, the positioning member holder comprises a longitudinal axis forming an angle comprised between 0° and 20° with the first guiding assembly longitudinal axis.

In other words, the positioning member holder is angled relative to the first guiding assembly longitudinal axis to enhance penetration and ensure a more secure grip of the second positioning member on the second bone portion.

According to one embodiment, the second guiding assembly comprises a positioning member support mounted on the carriage, the positioning member support comprising the first positioning element, the carriage being rotatable around a carriage axis in guide in such a way that a rotation of the carriage is configured to rotate in turn the first contact site.

Simply put, the first positioning element, housed in the positioning member support that is mounted on the carriage is rotatable around the carriage axis such that the second bone portion can be displaced out of the surgical plane, enabling adjustments in an additional direction.

According to one embodiment, the positioning member holder of the first guiding assembly and the positioning member support of the second guiding assembly are integrated into a single component comprising a common positioning member.

Stated otherwise, a single component can both move the second bone portion laterally in the surgical plane and outside of the surgical plane in a sensibly orthogonal trajectory. Combining both functions in one component reduces the complexity of the surgical guide, minimizing the number of moving parts and the risk of mechanical failure. It further reduces the overall size of the surgical guide, making it less intrusive and easier to handle during surgery.

According to one embodiment, the adjustment member comprises a third guiding assembly comprising a second positioning element configured to come in contact with the second bone portion at a second contact site, said second positioning element being configured to move the interaction point around a rotation axis, wherein said rotation axis is parallel with the body median plane, so as to move said second bone portion around a longitudinal central axis of said second bone portion.

Advantageously, the surgical guide according to the invention enables automatic positioning of the second bone portion (e.g., first metatarsal bone head) with respect to the first bone portion (e.g., first metatarsal bone base) in an additional direction beyond the lateral translation and the adjustments transverse to the median plane of the foot. The additional adjustment consists in the rotation of the second bone portion around its longitudinal central axis. This allows for more precise anatomical corrections, better alignment of the bone portions, and improved functional outcomes. Moreover, this additional rotational adjustment reduces the risk of residual deformities or misalignments, contributing to the overall stability and durability of the surgical results. The capability to rotate the bone portion also minimizes the need for intraoperative manual manipulations, which can reduce surgical time.

According to one embodiment, the second guiding assembly and the third guiding assembly share a common positioning element configured to come in contact with the second bone portion at a single contact site.

To put it differently, the sensibly transverse adjustments of the second bone portion and the rotation of the second bone portion may be obtained through a single positioning element. In the same manner, the transversal push and/or pull may also be obtained through the same positioning element.

According to one embodiment, the common positioning element is a wire configured to penetrate the second bone portion.

According to one embodiment, the third guiding assembly comprises a positioning member retainer rotatably mounted on the first guiding assembly so as to move the interaction point around said rotation axis.

According to one embodiment, the positioning member retainer is rotatably mounted on the carriage of the first guiding assembly.

According to one embodiment, the surgical guide comprises at least one marker configured to be detected by a surgical navigation system.

According to one embodiment, the surgical guide comprises a non-magnetic-field-disturbing material configured to be compatible with the surgical navigation system.

According to one embodiment, the surgical guide comprises at least one nonmagnetic-field-disturbing structure configured to be compatible with the surgical navigation system.

According to one embodiment, the first bone portion and the second bone portions are obtained by osteotomy of the first metatarsal bone during hallux valgus surgery.

According to another aspect, the invention pertains to a system comprising:
- the surgical guide according to any of the preceding embodiments, and
- a surgical navigation system.

According to another aspect, the invention pertains to a kit comprising:
- the surgical guide according to any of the preceding embodiments, and
- a set of fixing systems comprising wires.

According to another aspect, the invention related to a method for moving a second bone portion between a pathological position and an adjusted position with respect to a first bone portion, during osteosynthesis surgery, using a device according to any of the preceding embodiments, the surgical guide being configured to be positioned in a surgical plane of the first bone portion and second bone portion, said method comprising simultaneously moving the securing assembly between a first extremal position and a second extremal position to modify the fixing angle, and the first guiding assembly along the longitudinal axis, so that a displacement of the second bone portion along the longitudinal axis implies a modification of the fixing angle, ensuring the fixing wire can traverse both the first bone portion and the second bone portion after a displacement of the first guiding assembly.

The steps of adjusting the positioning member position and adjusting the positioning element position may be performed in this order or in a reverse order or simultaneously.

According to other aspects of the invention, the method comprises one or more of the steps described in the following embodiments, taken alone or in any possible combination.

According to one embodiment, said method further comprises adjusting a second positioning element position so as to move a second contact site around a rotation axis parallel with the body median plane, so as to move said second bone portion around a longitudinal central axis of said second bone portion.

### BRIEF DESCRIPTION OF THE DRAWINGS

**Figure 1** is a lateral view of the surgical guide according to one embodiment of the invention,
**Figure 2** is a lateral view of the surgical guide according to one embodiment of the invention, illustrating the fixing angle (β)
**Figure 3** is a top view of the surgical guide from figure 1 in a first position of the second guiding assembly, the surgical guide being positioned at the anchoring point in the first metatarsal bone head
**Figure 4** is a top view of the surgical guide from figure 1 in a second position of the second guiding assembly,
**Figure 5** is a top view of the surgical guide from figure 1 in a first position of the third guiding assembly,
**Figure 6** is a top view of the surgical guide from figure 1 in a second position of the third guiding assembly,
**Figure 7** is a schematic representation of a foot presenting a deformity of the first metatarsophalangeal (MTP) joint,
**Figure 8** is a schematic representation of the foot from figure 7, after osteotomy of the first metatarsal bone,
**Figure 9** is a schematic representation of the foot from figure 6, after adjusting the position of the first metatarsal bone head, and insertion of the fixing wires to secure the first metatarsal bone head with the first metatarsal bone base in the adjusted position.

### DETAILED DESCRIPTION

The present description illustrates the principles of the present disclosure. It will thus be appreciated that those skilled in the art will be able to devise various arrangements that, although not explicitly described or shown herein, embody the principles of disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, *i.e.,* any elements developed that perform the same function, regardless of structure.

The surgical guide 1000 is a medical device designed to assist surgeons in precisely executing osteosynthesis surgical procedures, by providing physical guidance for bones, tools and/or implants. The surgical guide 1000 notably helps align and stabilize bone portions obtained after an osteotomy and/or a bone fracture.

The surgical guide 1000 is preferably made of at least one biocompatible, durable and sterilizable material, suitable for use in an operating room. The choice of material depends on factors such as the intended application, required strength, and method of fabrication (e.g., 3D printing, injection molding, machining). Common materials include polymers (Medical-grade nylon (polyamide), Polyetheretherketone (PEEK), Acrylic or polymethylmethacrylate (PMMA), Polycarbonate), Polyphenyl sulfon (PPS) and/or metals (stainless steel, titanium), composite materials (Carbon fiber-reinforced polymers, Ceramic-polymer composites). To enhance functionality and safety surgical guide 1000 may include surface coatings such as antimicrobial coatings, hydrophobic or hydrophilic coatings and/or abrasion-resistant coatings).

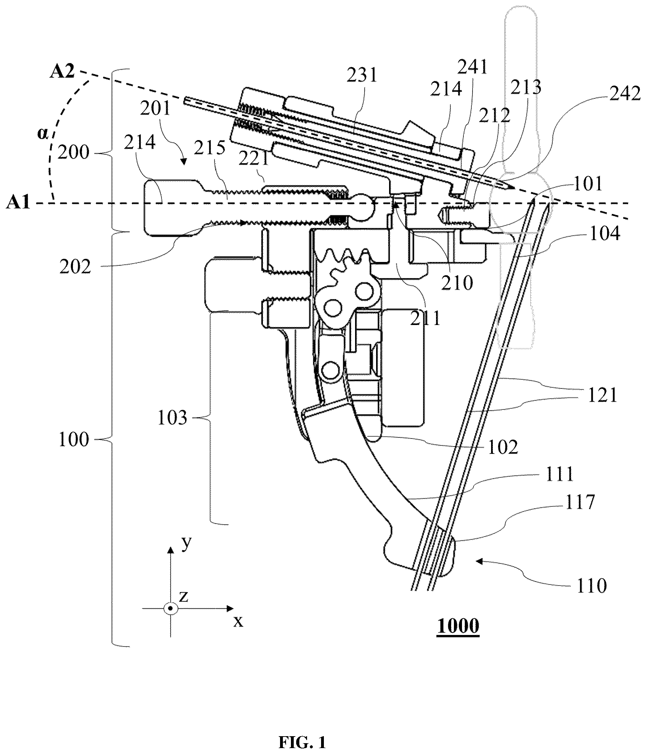

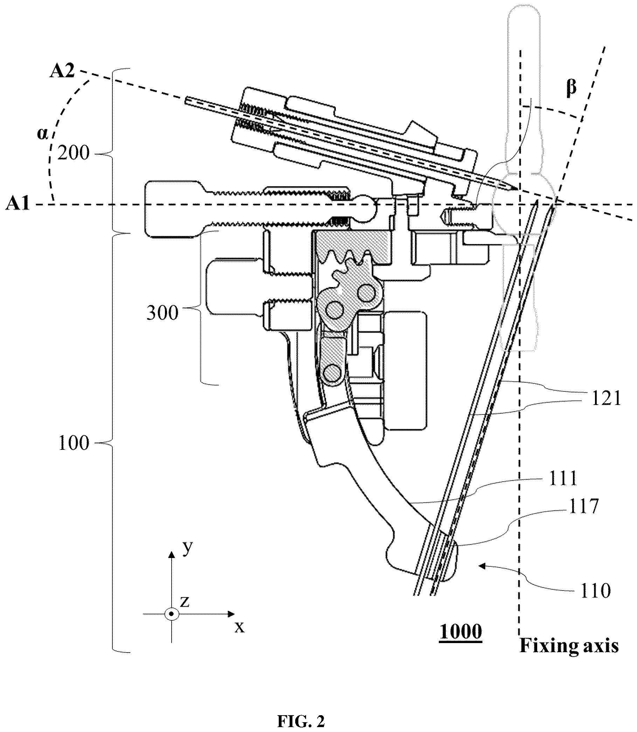

Referring to **Figure 1** and **Figure 2****,** the surgical guide 1000 according to the invention comprises a body 100, an adjustment member 200.

The adjustment member 200 comprises a plurality of guiding assemblies configured to manipulate at least one bone portion along distinct axes and orientations, enabling precise repositioning of the bone during surgical procedures. Typically, in the case of hallux valgus surgery, the guiding assemblies are configured to move a second bone portion (e.g. big toe and head of first metatarsal bone) between a pathological position and an adjusted position with respect to a first bone portion (e.g. first metatarsal bone base). At least the second bone portion is moved in an inward lateral translation in of the metatarsal head in a surgical plan, in order to reduce the hallux valgus.

A first guiding assembly is designed to translate the bone portion along a longitudinal axis (e.g., the x-axis) of the first guiding assembly by dragging or pushing the bone portion. The pushing and pulling may be performed by a same positioning member or by distinct positioning members. Typically, the pushing and pulling may be obtained using wires or forceps that anchor to the bone portion. Alternatively, the pushing may be performed by a pusher that comes in contact with the bone portion, while the pulling may be performed by a wire or forceps that anchor to the bone portion.

As illustrated on **Figure 1** and **Figure 2****,** the first guiding assembly 201 may include a carriage 210, which is movably mounted on the body 100 of the surgical guide 1000. Typically, the carriage 210 may be translatable within a groove 130 formed in the upper part of the body 100 (e.g., in the upper portion of the C-shaped body). Notably, the groove 130 longitudinal axis is oriented along the x-axis. The carriage 210 is equipped with a central axis 211, which is configured to be driven along the groove 130.

The translation of the carriage 210 along the groove 130 is achieved through a translation screw mechanism. The translation screw 215 is operably connected to a knob 214, which allows the surgeon to incrementally rotate the translation screw 215 manually. The translation screw 215 is threaded through a mechanical controller 221 that is rotatably fixed within the body 100 of the surgical guide 1000. This configuration ensures that when the knob 214 is rotated, the translation screw 215 remains axially fixed (e.g., parallel to the longitudinal axis A1 of the first guiding assembly 201) within the mechanical controller 221, translating rotational motion into controlled linear displacement of the carriage 210.

The proximal end (proximal with respect to the bone portion) of the translation screw 215 is embedded in the carriage 210. As the translation screw 215 rotates within the mechanical controller 221, its threads engage with the mechanical controller 221's internal threading, causing the translation screw 215 to advance or retract along the longitudinal axis A1. This linear motion, in turn, pushes or pulls the carriage 210 within the groove 130.

At its proximal extremity (proximal with respect to the bone portion), the carriage 210 features a first positioning member (e.g., pusher 212) that is configured to come into direct contact with the bone portion at a first contacting point 213, enabling controlled translation of the bone portion along the longitudinal axis A1 of the first guiding assembly 201. The first positioning member may be monolithic with the carriage 210 (e.g., for instance 3D printed or molded in a single bloc) or removably fixable to the carriage 210, for instance using a locking mechanism (e.g., screwed into a bore formed in the carriage 210), welding or bonding etc.

Notably, in **Figure 1** and **Figure 2****,** when the carriage 210 is translated using the translation screw 215 actuated by the knob 214, the pusher 212 comes into contact with the bone portion and exerts a pushing force.

The body 100 extends in a median plane parallel to the xOy plane and features a bone anchoring member 101 and a securing assembly 110. Notably, the median plane is chosen as the plane parallel to the xOy plane that comprises the anchor point 104 of the bone anchoring member 101 destined to come in contact with a bone portion.

As illustrated on **Figure 1** and **Figure 2****,** the body 100 may haves a curved configuration, generally C-shaped configuration, designed to facilitate secure handling and precise surgical operations, with a height (along axis y) comprised between 5 cm and 20 cm, a width (along axis x) comprised between 2 cm and 10 cm and a thickness (along axis z) comprised between 1 cm and 6 cm.

The body 100 may comprise a griping portion 103 configured to fit comfortably in the surgeon's hand, for instance located on the curved section of the "C". This portion may feature textured surfaces, such as ridges or rubberized coatings, to enhance grip and prevent slipping during the procedure.

As mentioned above, the bone anchoring member 101 is configured to secure the surgical guide 1000 to a bone portion (e.g., the inferior portion of the first metatarsal bone in a hallux valgus surgery) at an anchor point 104. The bone anchoring member 101 may be positioned at the upper extremity of the body 100, and notably at the upper extremity of the "C".

The bone anchoring member 101 may notably be configured to be inserted into the exposed inside part of the bone portion (e.g., exposed due to an osteotomy or a bone fracture), to engage the cortical bone, which is located on the outer surface of the bone portion. The bone anchoring member 101 may further be configured to prevent rotational movement, keeping the surgical guide 1000 properly aligned in relation to the bone portion and the osteotomy site.

In **Figure 1****,** the bone anchoring member 101 is a hook, comprising a sharp or curved end forming the anchor point 104. The hook might have a barbed or serrated structure that allows it to "bite" or lock into the bone portion, preventing it from slipping out. Once the hook is in place, the force from the surgical guide and the soft tissue's natural tension will press the hook against the bone portion, holding it securely in position.

Alternatively, the bone anchoring member 101 may comprise several hooks and/or at least one wire, such as a Kirschner Wires (thin, flexible stainless-steel pins), at least one screw, at least one pin, and/or at least one pliers. The bone anchoring member 101 may be monolithic with the body 100 (e.g., 3D printed or molded in a single bloc) or removably fixable to the body 100, for instance using a locking mechanism, welding or bonding etc. The bone anchoring member 101 may be inserted in the bone portion manually or with the assistance of a tool (e.g., a surgical hammer, a trocar, a bone awl, a pin driver, a drill etc.). The bone anchoring member 101 may typically be placed in such a way that its curved or pointed portion penetrates the bone slightly (the penetration should be minimal to void damage, e.g., in hallux valgus surgery, the hook may penetrate the bone by a distance comprised between 1 cm and 5 cm).

The bone anchoring member 101 can be a temporary fixture during the surgical procedure, used just to stabilize the surgical guide 1000. After completing the surgery, the bone anchoring member 101 may be removed, leaving the surgical site prepared for permanent fixation if needed.

The body 100 also features a securing assembly 110 configured for the precise adjustment of the position and orientation of fixation wires 121 (e.g., K-wires), a drill and/or screws configured to be inserted in the bone portions. The securing assembly 110 is configured to operate by translating or rotating specific components within a constrained range of motion, allowing the fixation wires 121 (e.g., K-wires), drill and/or screws to move between two predefined extremal positions.

As illustrated on **Figure 1****,** the securing assembly 110 includes a guiding piece 111 comprising at least one hole 117 through which fixation wires (e.g., K-wires), a drill and/or screws can be inserted.

As illustrated on **Figure 1** **and** **Figure 2****,** the securing assembly 110 is adjustable between two predeterminate positions enabling to adjust a fixing angle (β) defined by the fixing wires (e.g., the longitudinal axis of the fixing wires) and a first bone portion central axis tangential to the anchoring point. The two predetermined positions are a first extremal position and a second extremal position predetermined along the body 100.

The body 100 may be in a curved configuration enabling the securing assembly 110 to be adjusted along the body 100. Typically, the range of movement enabled by the curved configuration is configured to adjust the fixing angle (β) between -20° and 30°.

As illustrated on **Figure 1** **and** **Figure 2****,** the securing assembly 110 and the first guiding assembly 201 are cinematically synchronized by a coupling member 300. In other words, the fixing angle (β) is modified according to the movement of the positioning member 212, 241, during the adjustment of the position of the second bone portion 12a.

A first embodiment may involve a rotation of the translation screw 215 within the mechanical controller 221. As the translation screw 215 is embedded in the carriage 210, the rotation of the translation screw 215 will cause the carriage 210 to advance or move back along the x-axis (A1).

Alternatively, the translation screw could be replaced by a piston, a slider or a motor.

In a preferred embodiment, the coupling member 300 is in relation with the first guiding assembly 201 thanks to a gear rack 301, transforming the translation of positioning member along the x-axis (A1) into a rotation of the a mechanical transmission 302 (e.g a gear wheel) meshed with the gear rack 301. The mechanical transmission 302 is linked to a rod 303 in relation with the securing assembly 110. By consequence, a translation of the first guiding assembly 201 along the y-axis of the median plane implies a modification of the angle (β).

The dimensions of the kinematics elements linking the first guiding assembly to the securing assembly 110 are adapted so that the modification of the angle (b) matches the displacement of the second bone portion, in order to guarantee the wire 121 will traverse both the first bone portion and the second bone portion.

For example, the length of the rod 303, as well as the teeth modulus of gear rack 301 and the mechanical transmission 302 can be adapted. In some embodiments, there is no rod 303 and the securing assembly 110 is directly linked to the mechanical transmission 302.

In other non-illustrated embodiments, the kinematic relation between the first guiding assembly 201 and the securing assembly 110 comprises a belt, or a chain, or an elastic part, or an actuator whose displacement is regulated according to the position of the first guiding actuator 201.

The translation of the rod 303 is configured to adjust the position of the securing assembly between the first extremal position and the second extremal position.

The coupling member 300 may be reversible and enable a movement of the securing assembly 110 in both directions, that is to say the securing assembly 110 can be adjusted from a first extremal position to a second extremal position, and from a second extremal position to a first extremal position , while the first extremal position correspond to a minimal position of the first guiding assembly 201 and the second extremal position correspond to a maximal position of the first guiding assembly. In other word, a rotation clockwise or counter clockwise of the translation screw 215 enables to respectively reduce or increase the fixing angle (β).

Alternative embodiment of the coupling member may involve a gear belt, embedded micromotors, or any mechanical solution enabling a cinematic transformation. Alternative embodiment may also consider material flexibility.

The coupling member may include a locking function enabling to secure the fixing wire position and prevent from uncontrolled movement which could affect the position of the securing assembly. This locking function can be provided by a protective pad 305 localized against a cam 304 of the mechanical transmission 302. As the mechanical transmission 302 meshes with the gear rack 301, the protective pad 305 is compressed between the cam 304 and the rod 303. Once the protective pad 305 is compressed, frictional forces against the cam 304 will prevent any uncontrolled movement of the securing assembly 110.

In other words, the actuation of the translating screw controls the coupling member 300 and its position cannot be modified without changing the position of the positioning member.

Another embodiment of the locking function can be provided by the helicoidal assembly of the translation screw 215 and the mechanical controller 221, which are made of materials and present a thread angle adapted to render the helicoidal assembly non-reversible. The securing assembly 110 can be displaced only if the translation screw is 215 is rotated, thus preventing the securing assembly to move without actuation of the first guiding assembly 210.

The mechanism provided by the coupling member 300 allows the surgeon to precisely adjust the position and angle of the fixation wires or screws, ensuring accurate placement relative to the bone portions in a single action being the inward translation of the second bone portion. The carriage 210 further comprises a window 214 formed in its upper portion, designed to securely accommodate a cylinder 231 (i.e., positioning member holder).

The window 214 is dimensioned and shaped to hold the cylinder 231 in such a way that the longitudinal axis A2 of the cylinder 231 forms an angle α comprised between 0° and 40° with the longitudinal axis A1. Advantageously the angle α is comprised between 0° and 30°, more advantageously the angle α is comprised between 0° and 20°. To ensure stability and alignment, the cylinder 231 may be retained within the window 214 using lateral retaining structures or bearings. These retaining elements maintain the position of the cylinder 231 relative to the carriage 210.

The cylinder 231 houses a wire 241 (i.e., second positioning member) that extends through its internal channel. This wire 241 is configured to engage with the bone portion at a second contacting point 242. As the carriage 210 translates, the bone portion is pulled accordingly.

A second guiding assembly 202 is configured to translate the bone portion generally along the z-axis, enabling movement in both directions of the z-axis. The translation can be either linear or arcuate, depending on the mechanism's design and operation. Overall, the bone portion is adjusted between an initial position and a final position respectively comprised in a first orthogonal plane and a second orthogonal plane, both parallel to the median plane (or yOz plane). The final position may either directly be the adjusted position (in case the second bone portion does not need angular adjustment and/or adjustment in the surgical plane) or a revised position wherein the second bone portion 12a is comprised outside the surgical plane.

The movement between these positions is allowed by either a single first positioning element or distinct first positioning elements, depending on the configuration. Typically, this pushing and pulling action may be achieved through the use of wires or forceps, which anchor to the bone portion to apply the necessary forces for translation. The first positioning element may have other functions than the translation of the bone portion generally along the z-axis, such as the translation of the bone portion along another axis, such as the longitudinal axis (e.g., the x-axis) or the rotation of the bone portion around its central longitudinal axis.

As illustrated on **Figure 1** and **Figure 2****,** the second guiding assembly 202 may comprise the carriage 210 that is rotatable around its central axis 211. The rotation of the carriage 210 is driven by the rotation of the mechanical controller 221, that is actionable by the surgeon. The mechanical controller 221 rotates around a central axis of the mechanical controller, rotatably embedded in the body 100. The rotation of the mechanical controller 221 transmits its rotational movement to the carriage 210 via a ball joint 216 that connects the mechanical controller 221 to the carriage 210.

The cylinder 231 (i.e., positioning member support) held by the carriage 210 houses a wire 241 (i.e., first positioning element) that extends through its internal channel. This wire 241 is configured to engage with the bone portion at a first contact site 242. As the carriage 210 translates, the bone portion is pulled accordingly.

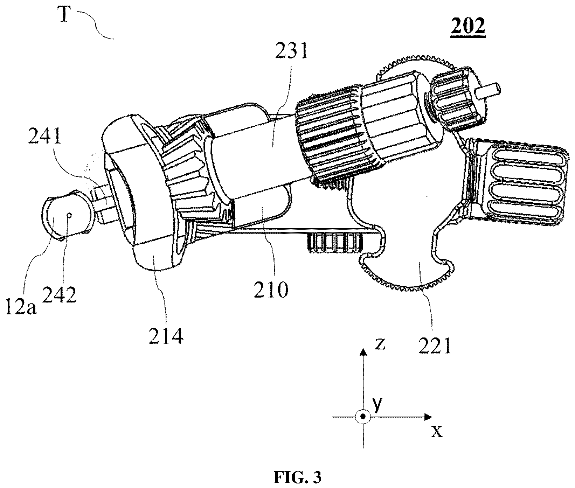

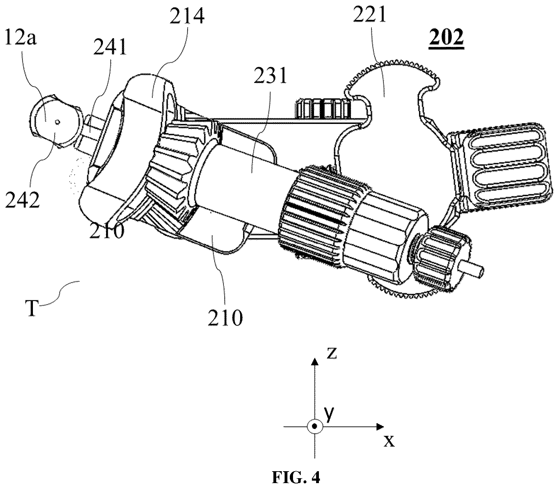

Thus, as illustrated on **Figure 3** and **Figure 4****,** the contact site 242 has a controlled arc-like trajectory T along a curved path generally oriented along the z-axis and whose central axis is the carriage 210 central axis 211. This movement results in the bone portion 12a being pushed or pulled along the trajectory T. Typically, the bone portion 12a may cover a trajectory comprised between -20° and 20°.

A third guiding assembly 203 enables angular rotation of the bone portion around its central longitudinal axis (parallel to y-axis). The angular rotation may be performed by a second positioning element (e.g., wires or forceps). Typically, the angular rotation may be comprised between -30° and 30°. The second positioning element may have other functions than the rotation of the bone portion, such as the translation of the bone portion along another axis, such as the longitudinal axis (e.g., the x-axis) or the translation of the bone portion outside of the surgical plan.

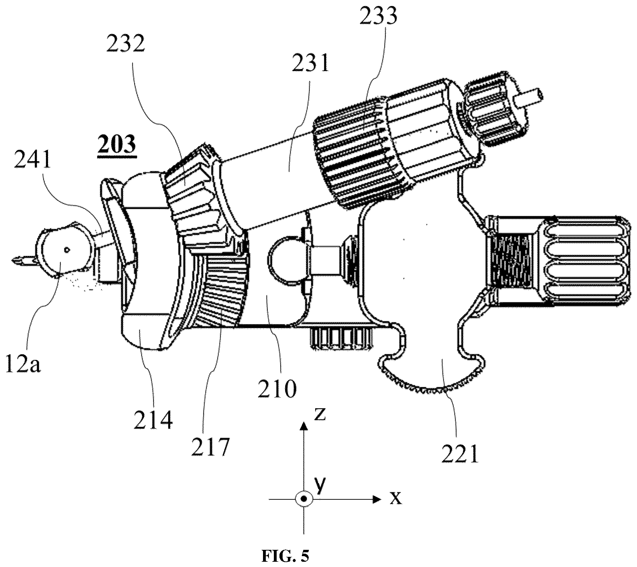

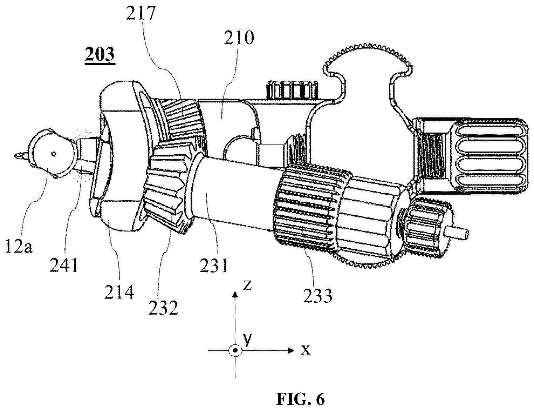

As illustrated on **Figure 1** and **Figure 2****,** this functionality is achieved through the wire 241 housed within the cylinder 231 (i.e., second positioning element) mounted on the carriage 210. As already mentioned, the cylinder 231 is positioned in a window 214 located at the top of the carriage 210. As illustrated on **Figure 5** and **Figure 6****,** the cylinder 231 incorporates a pinion 232 that engages with a rack 217 formed on the carriage 210, allowing the cylinder 231 to rotate within the window 214 when actuated. Actuation may be performed manually by the surgeon, notably using the gripping ring 233 positioned on the cylinder 231. This rotation of the cylinder 231 imparts a rotational force to the wire 241 (i.e., second positioning element), which, being anchored to the second bone portion 12a, causes the second bone portion 12a to rotate about its central axis.

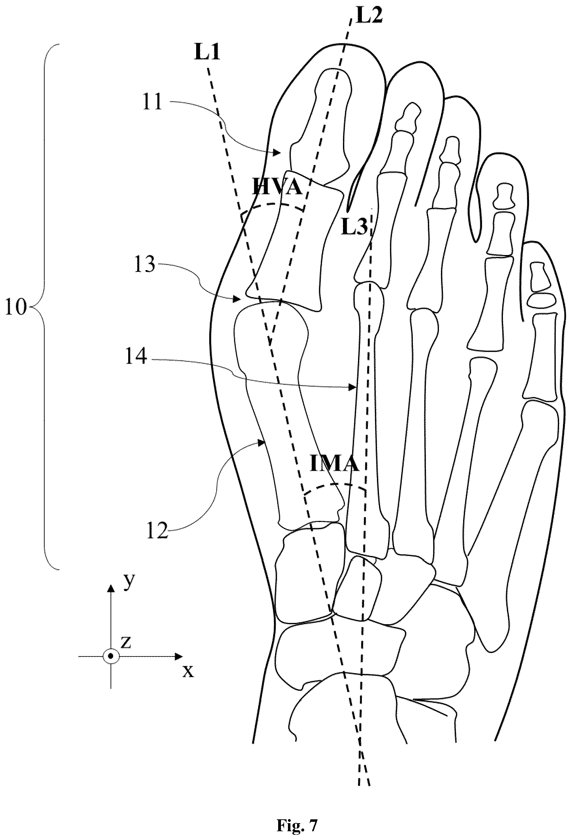

As illustrated on **Figure 7****,** the foot (e.g. human foot) comprises a first ray 10 encompassing the first metatarsal bone 12 and the big toe 11 (also called "hallux"). The first metatarsal bone 12 and the big toe 11 are articulated around the first metatarsophalangeal (MTP) joint 13.

A hallux valgus is a progressive deformity of the first MTP joint 13, characterized by a deviation (also called "valgus") of the big toe 11 and of the first metatarsal bone 12. This condition leads to the development of a bony prominence, or bunion, on the medial aspect of the foot.

Clinically, a hallux valgus may be described using the hallux valgus angle (HVA) and the intermetatarsal angle (IMA).

The HVA quantifies the lateral deviation of the big toe 11 relative to the first metatarsal bone 12. It is formed between a longitudinal axis of the first metatarsal bone L1 and a longitudinal axis of the big toe L2. A normal HVA is generally inferior to 15°. Mild Hallux Valgus generally presents a HVA comprised between 15° and 20°, moderate Hallux Valgus generally presents a HVA comprised between 20° and 40°, while severe Hallux Valgus present a HVA superior to 40°.

The IMA measures the deviation of the first metatarsal 12 relative to the second metatarsal bone 14. It is formed between the longitudinal axis of the first metatarsal bone L1 and a longitudinal axis of the second metatarsal bone L3. A normal IMA is generally inferior to 9°. Mild Hallux Valgus generally presents an IMA comprised between 9° and 11°, moderate Hallux Valgus generally present an IMA comprised between 11° and 16°, while severe Hallux Valgus presents an IMA superior to 16°.

Osteosynthesis surgery involves several steps, some of which may be optional depending on the specific requirements of the surgery and the patient's condition. Additionally, the steps may be performed in the following order or in a different order without changing the invention. The method may be divided into preoperative and intraoperative steps, with various alternative embodiments depending on the surgical approach and tools used.

The first optional step is the imaging of the anatomical structure to be operated on (e.g. the foot and particularly the first metatarsal and the big toe in case of hallux valgus surgery) preoperatively, in order to obtain a 3D reconstruction of the anatomical structure. Various imaging techniques can be employed to capture 2D images (e.g. at least two 3D images and preferably three 2D images), such as X-ray, CT scans, or MRI, or alternatively direct 3D imaging systems such as Cone Beam Computed Tomography (CBCT), 3D Ultrasound Imaging, or Optical 3D Scanning. The at least two 2D images may be captured from different angles to facilitate the reconstruction of a 3D image of the target anatomical structure and more precisely of the bones comprised in the target anatomical structure. The 3D image obtained from the previous steps can then be segmented to isolate the bone structures and create a 3D preoperative model of at least the bone structures present in the target anatomical structure. Generally, the imaging technique employed preoperatively allows a better visualization of the target anatomical structure than imaging techniques available intraoperatively. The 3D preoperative model obtained preoperatively allows the surgeon to plan the surgical procedure in detail, notably by determining the extent of the bone deformity/misalignment, the optimal osteotomy location, and the desired post-surgical alignment. For instance, in the case of a hallux valgus surgery, the ideal angles, such as the intermetatarsal angle (IMA) and/or hallux valgus angle (HVA) maybe planned on the 3D preoperative model.

The second optional step is the imaging of the anatomical structure to be operated on, intraoperatively. Such imaging may be performed using fluoroscopy, intraoperative computer tomography, intraoperative MRI, a C-arm, ultrasound imaging or optical tracking (e.g., camera). Intraoperative imaging provides real-time data on the actual position and orientation of the anatomical structures during surgery. It accounts for variations caused by patient positioning, tissue deformation, or movement, which cannot be predicted during the preoperative phase.

The third optional step involves registering the preoperative 3D model with the at least one intraoperative image. This step ensures that the actions planned preoperatively on the preoperative 3D model are accurately mapped to the patient's anatomy and position during surgery. Registration methods such as marker-based registration or anatomical landmark registration, iterative closest point (ICP) registration or a combination thereof may be used.

Optionally, markers may be used to help with registration of the preoperative 3D model and the least one intraoperative image. To that end, markers (e.g., electromagnetic trackers (EM trackers), optical trackers, or ultrasound-based markers) may be positioned and/or implanted into the target anatomical structure. These markers are typically placed at key locations on the target anatomical structure that will facilitate registration. For instance, in the case of hallux valgus surgery, EM trackers may be implanted in the first metatarsal bone, for instance as the base of the first metatarsal bone and the head of first metatarsal bone, on both sides of the future osteotomy.

Additionally, a navigation system may be used during the surgery. It provides to the surgeon a visual representation of the surgical field in real time, guiding the surgeon through the procedure with enhanced precision. It integrates data from preoperative planning, intraoperative imaging, and a tracking system to display the position of anatomical structures, surgical instruments, and implants relative to each other. The primary goals are to improve accuracy, minimize complications, and ensure adherence to the surgical plane.

The tracking system is configured to monitor the precise position and orientation of anatomical structures (e.g., bones, cartilages, ligaments etc.), surgical instruments (e.g. surgical saw, surgical guide etc.), and implants (e.g., screws, plates, wires etc.) in real time. Its purpose is to enhance surgical accuracy, ensure alignment with preoperative plans, and reduce errors, particularly in complex surgical procedures. The tracking system may be marker-less. In that case, the tracking system does not rely on physical markers to track the position of anatomical structures, tools, or devices. Instead, it uses other technologies that rely on imaging, computer vision, or other sensor-based methods to track movements or positions. Alternatively, the tracking system may rely on the same set of markers as the one used for registration or on a different set of markers positioned and/or implanted in the target anatomical structure. When relying on markers, the tracking system may be an optical tracking system that relies on a camera to track the markers or an electromagnetic tracking system that uses EM fields to track EM trackers embedded in the anatomical structure and/or in the surgical guide.

Based on the registration, the surgical plan is adapted to the specificity of the surgical set (e.g., position of the patient, surgical instruments used, room and material specificity etc.). The adaptation may be performed automatically by the navigation system or manually by the surgeon.

The navigation system then provides the necessary steps and adjustments to achieve the desired adjusted position. In the case of hallux valgus surgery, the navigation system may provide the correct IMA (typically around 9°), as well as the correct HVA. This may translate into recommendations and/or instructions for the surgical guide (e.g., rotation of the knobs, mechanical controller and/or cylinder in the window).

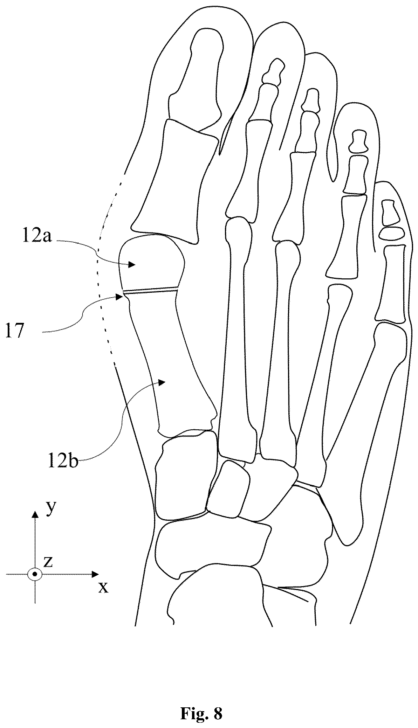

The next optional step involves performing the osteotomy of the bones to be operated on (this step may not be necessary in case of a bone fracture setting). The osteotomy step involves cutting at least one bone into at least two bone portions. In the case of hallux valgus surgery, illustrated on **Figure 8****,** the osteotomy step involves cutting the first metatarsal bone into a first bone portion 12a (first metatarsal bone head) and a second bone portion 12b (first metatarsal bone base). Various types of osteotomies may be used, depending on the severity of the deformity and the surgical approach. Common osteotomy techniques include distal osteotomy, wherein a transversal cut is made closer to the head of the metatarsal, proximal osteotomy, wherein a transversal cut is made closer to the base of the metatarsal, chevron osteotomy, wherein a V-shaped cut is performed, etc. The osteotomy can be performed manually by the surgeon using a surgical saw or osteotome, or it may be assisted by automated tools.

A surgical plane can be established as the median plane relative to the different bone portions. Indeed, the bone portions may exhibit irregular positioning or misalignment in space due to deformation or displacement resulting from a fracture. The median plane may therefore be inclined with respect to the xOy plane.

The next step involves the installation of the surgical guide with respect to the target anatomical structure. For hallux valgus surgery, as visible on **Figure 1****,** the surgical guide 1000 is generally placed laterally to the foot (e.g., with the median plane of the body 100 parallel to the xOy plane). Alternatively, the surgical guide 1000 may be positioned on other positions, such as on top of the foot, depending on the surgical approach and the configuration of the target anatomical structure.

The surgical guide 1000 may be positioned with the hook 101 anchored to the first metatarsal bone base 12b and the pusher 212 in contact with the first metatarsal bone head at a first contacting point 213. Moreover, a wire 241 can be inserted into the first metatarsal bone head through the cylinder 231, for example using a surgical hammer, or after drilling a hole into the first metatarsal bone head 12a.

After positioning the surgical guide, the surgeon can adjust the bone portions between the pathological position and the adjusted position by manipulating the adjustment means (e.g., knob 214, mechanical controller 221, cylinder 231 position in the window 214) of the surgical guide 1000. For this purpose, the surgeon can follow the recommendations and/or instructions provided by the navigation system.

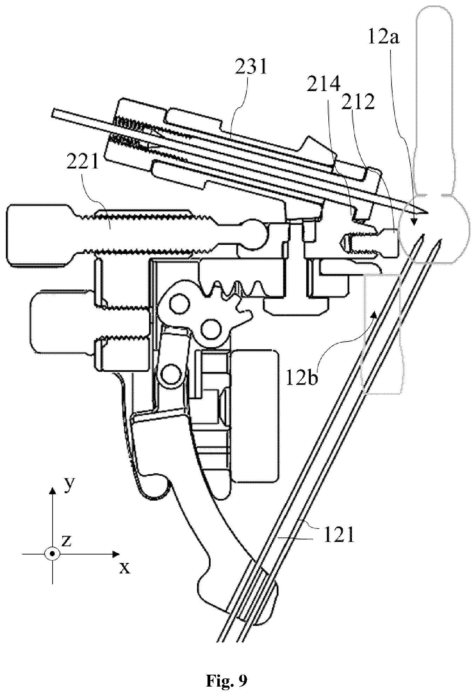

For instance, the surgeon may first rotate the cylinder 231 around the central axis of the carriage 210 to position the pusher on the first metatarsal bone head 12a and then linearly push the first metatarsal bone head 12a, as illustrated on **Figure 9****.** The surgeon may then adjust the first metatarsal bone head 12a height (i.e., position along z-axis) by rotating the mechanical controller 221 and finally adjust the rotation of the first metatarsal bone head 12a by moving cylinder 231 in window 214. However, another order may be contemplated without changing the scope of the invention.

Once the adjusted position is achieved, as illustrated on **Figure 9****,** the surgeon may use wires 121 to temporarily fix the bones portions 12a, 12b, for example using a surgical hammer, by insertion after drilling a hole into the first metatarsal bone head 12a.

This step is followed by the insertion of fixing elements 16 (e.g., screws, self-tapping screws, cannulated screws, locking screws, absorbable screws, plates, tension band wirings etc.), to stabilize the osteotomy site or fracture site. Finally, osteogenesis 15 and/or osteointegration occurs through a series of natural biological processes that enable the bone to regenerate and restore its original structure and function.

## Claims

1. A surgical guide (1000) for moving a second bone portion (12a) between a pathological position and an adjusted position with respect to a first bone portion (12b), during osteosynthesis surgery, the surgical guide (1000) being configured to be positioned in a surgical plane of the first bone portion (12b) and the second bone portion (12a), said surgical guide (1000) comprising:
• a body (100) with an upper extremity (100a) and a lower extremity (100b), extending in a median plane, and comprising
∘ a bone anchoring member (101) configured to be in contact with the first bone portion (12b) at an anchor point (104) in the surgical plan,
∘ a securing assembly (110) comprising at least one fixing wire (121), said at least one fixing wire (121) being configured to be inserted into the first bone portion (12b) and the second bone portion (12a) to secure the first bone portion (12b) with the second bone portion (12a), once the second bone (12a) portion is in the adjusted position, the fixing wire being inclined of a fixing angle (β) with relation to a central axis of the first bone portion,
▪ the fixing angle (β) being measured in the surgical plane between the fixing wire and the central axis of the first bone portion and,
▪ the central axis being tangential to the anchoring point (104),
∘ the fixing angle (β) being adjustable between a first extremal position and a second extremal position in order to set the direction of the at least one fixing wire (121) in relation to the second bone portion (12a),
• an adjustment member (200) comprising a first guiding assembly (201) connected to the upper extremity (100a) of the body and moveable along a longitudinal axis (A1) of the median plane, wherein the first guiding assembly (201) includes one positioning member (212, 241) configured to come in contact with the second bone portion (12a) in at least one contacting point (213, 242) to move the second bone portion along the longitudinal axis (A1),
***characterized in that*** the securing assembly (110) is connected to the first guiding assembly (201) by a coupling member (300) configured to simultaneously move :
- the securing assembly (110) between the first extremal position and the second extremal position to modify the fixing angle (β) and
- the first guiding assembly (201) along the longitudinal axis (A1),
so that a displacement of the second bone portion along the longitudinal axis implies a modification of the fixing angle (β), ensuring the fixing wire can traverse both the first bone portion and the second bone portion after a displacement of the first guiding assembly.

2. The surgical guide according to claim 1, wherein the body (100) extends from the upper extremity towards the lower extremity according to a curvature parallel to the surgical plane, and the securing assembly (110) is movable along the curvature between the first extremal position and the second extremal position.

3. The surgical guide according to any one of claims 1 or 2, wherein the coupling member (300) is configured to be locked when the first guiding assembly (201) is not actuated.

4. The surgical guide according to claim 3 wherein the first guiding assembly comprises a translation screw (215) threaded through a mechanical controller (221) that is fixed to the body and wherein the assembling of the translation screw (215) and the mechanical controller (221) is configured to present a tightening torque preventing uncontrolled movements.

5. The surgical guide according to any one of claims 1 to 4, wherein the coupling member (300) allows the securing assembly (110) to be reversibly adjusted, in a direction from the first extremal position to the second extremal position , and in a reversed direction from the second extremal position to the first extremal position.

6. The surgical guide according to any of claims 1 to 5, wherein the coupling member (300) comprises a gear rack (301) connected with the first guiding assembly (201) and a mechanical transmission (302) which is meshed with the gear rack (301), and which is configured to be connected with the securing assembly (110) so that the the fixing angle (β) is adjusted according the meshing of mechanical transmission (302) when the gear rack (301) moves.

7. The surgical guide according to claim 6, wherein the mechanical transmission (302) comprises a cam configured to:
- face a pad linked to the securing assembly (110), when the securing assembly (110) is in the first/second extremal position,
- come into contact with the pad, when the securing assembly (110) is in an intermediate position between the first extremal position and the second extremal position,
- apply a pressure on the pad when the securing assembly (110) is in the second /first extremal position.

8. The surgical guide according to claim 6 or 7, wherein the cam (304) of the mechanical transmission (302) is in connection with the securing assembly (110) through a connecting rod (303) configured to adjust the fixing angle (β) according to the mechanical transmission (302) rotation.

9. The surgical guide according to claim 7 in combination with claim 8, wherein the protective pad (305) is mounted on the connecting rod (303).

10. The surgical guide according to any one of claim 1 to 9 wherein the adjustment member (220) further comprises a second guiding assembly (202) comprising a first positioning element (242) configured to come in contact with the second bone portion (12a) at a first contact site (243), the first positioning element (242) being configured to move the contact site (243) from an initial position in the surgical plane to a revised position comprised in a plane orthogonal to the body median plane and wherein in the revised position, the second bone portion (12a) is comprised outside the surgical plane.

11. The surgical guide according to claim 10, wherein the first guiding assembly (201) comprises a carriage (210) movable along a guide (130) formed in the body (100) following the longitudinal axis (A1), said carriage (210) comprising said at least one positioning member (212, 241).

12. The surgical guide according to claim 11, wherein the first guiding assembly (201) comprises a first positioning member (212) mounted on a lateral wall of the carriage (210), said first positioning member (212) being configured to push said second bone portion (12a) along the longitudinal axis (A1),

13. The surgical guide according to claim 11 or 12, wherein the first guiding assembly (201) comprises a positioning member holder (231) mounted on the carriage (210), said positioning member holder (231) comprising a second positioning member (241) configured to push and pull the second bone portion (12a) along the longitudinal axis (A1),

14. The surgical guide according to claim 13, wherein the positioning member holder (231) comprises a longitudinal axis (A2) forming an angle (α) comprised between 0° and 40 with the first guiding assembly longitudinal axis (A1),

15. The surgical guide according to claim 14, wherein the second guiding assembly (202) comprises a positioning member support (231) mounted on the carriage (210), the positioning member support (231) comprising the first positioning element (242), the carriage (210) being rotatable around a carriage axis (211) in guide (130) in such a way that a rotation of the carriage (210) is configured to rotate in turn the first contact site (243),
